(19)

Europäisches Patentamt
European Patent Office
Office européen des brevets

(11) **EP 4 748 308 A1**

(12) **EUROPEAN PATENT APPLICATION**

(43) Date of publication:
**27.05.2026 Bulletin 2026/22**

(21) Application number: **25216878.6**

(22) Date of filing: **19.11.2025**

(51) International Patent Classification (IPC):
***A61B 5/145*** (2006.01)

(52) Cooperative Patent Classification (CPC):
**A61B 5/14532**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **19.11.2024 CN 202411657184**

(71) Applicants:
• **Jiangsu Yuwell POCTech Biotechnology Co., Ltd.**
  **Zhenjiang, Jiangsu 212300 (CN)**
• **Zhejiang Poctech Co., Ltd.**
  **Huzhou, Zhejiang 313001 (CN)**
• **Jiangsu Yuekai Biotechnology Co., Ltd.**
  **Nanjing, Jiangsu 210018 (CN)**

(72) Inventors:
• **GUANQUN, Zhang**
  **Nanjing, Jiangsu 210018 (CN)**
• **YUCHUN, Min**
  **Nanjing, Jiangsu 210018 (CN)**
• **XIAOYU, Zhang**
  **Nanjing, Jiangsu 210018 (CN)**
• **MCCANLESS, Jonathan**
  **Nanjing, Jiangsu 210018 (CN)**
• **YANAN, Zhang**
  **Nanjing, Jiangsu 210018 (CN)**

(74) Representative: **Winter, Brandl - Partnerschaft mbB**
  **Alois-Steinecker-Straße 22**
  **85354 Freising (DE)**

(54) **COMPENSATION METHOD AND DEVICE FOR CONTINUOUS GLUCOSE MONITORING APPARATUS**

(57) The present invention discloses a compensation method and device for a continuous glucose monitor, belongs to the technical field of compensation for early signal attenuation of CGMs, and is used for resolving a technical problem that early signal attenuation affects stability and accuracy of the continuous glucose monitor (CGM) such that a user cannot obtain accurate glucose data at an early stage of using the CGM. The method includes: acquiring a real-time glucose value of a user continuously; determining a user type and a glucose trend type according to the real-time glucose value, where the user type includes at least a first user type and a second user type; determining, based on the real-time glucose value, whether a compensation start condition is satisfied; starting a compensation algorithm if the compensation start condition is satisfied; determining a compensation formula based on the real-time glucose value, the glucose trend type, and the user type; and obtaining, based on the real-time glucose value and the compensation formula, a compensated glucose value until a compensation stop condition is satisfied.

| | |
|---|---|
| Acquire a real-time glucose value of a user continuously | S101 |
| Determine a user type and a glucose trend type according to the real-time glucose value, where the user type includes at least a first user type and a second user type | S102 |
| Determine, based on the real-time glucose value, whether a compensation start condition is satisfied, and start a compensation algorithm if the compensation start condition is satisfied | S103 |
| Determine a compensation formula based on the real-time glucose value, the glucose trend type, and the user type | S104 |
| Obtain, based on the real-time glucose value and the compensation formula, a compensated glucose value until a compensation stop condition is satisfied | S105 |

FIG. 1

EP 4 748 308 A1

## Description

### TECHNICAL FIELD

The present invention relates to the technical field of compensation for early signal attenuation of continuous glucose monitoring apparatuses (CGM), and particularly relates to a compensation method and device for a continuous glucose monitor.

### BACKGROUND

As a small soft aseptic glucose monitoring sensor implanted under the skin of a user, a continuous glucose monitoring apparatus (CGM) can continuously monitor a glucose concentration in blood between subcutaneous tissue of the user. Moreover, a real-time glucose reading is transmitted to a mobile phone through Bluetooth, to display a fluctuation of a glucose level of the user to provide important diagnosis and treatment bases for the user and a doctor.

After the CGM is implanted under the skin of the user, due to damage to skin and/or tissue, different levels of rejections, anaphylaxis, or infected wounds may occur and cause a change in an environment of the CGM. The CGM is greatly affected by an environmental change. Common influence factors include a temperature, a detected glucose concentration, and the like. The environmental change generally causes attenuation of a detection signal of the CGM. Moreover, false hypoglycemia that a glucose concentration measured by the CGM is lower than an actual glucose concentration may occur. The false hypoglycemia generally occurs at an early stage of using the CGM by the user, generally lasts for several days, and subsequently gradually disappears. This phenomenon is referred to as early signal attenuation (ESA).

When the ESA occurs, an operating current of the CGM fluctuates, and a glucose value outputted by the CGM also fluctuates consequently. However, an actual glucose value of a human body does not change. Thus, an estimated glucose value largely deviates from the actual glucose value of the human body. It can be seen that the ESA has a significantly negative impact on stability and accuracy of the CGM. The user cannot obtain accurate glucose data at the early stage of using the CGM, and cannot timely and accurately determine the glucose level. Thus, it is urgent to suppress and compensate for the ESA, to reduce an occurrence probability of the ESA and improve accuracy of monitoring glucose by the CGM.

### SUMMARY

The present invention is set out in appended set of claims. The present invention provides a compensation method and device for a continuous glucose monitor, which are used for resolving a technical problem that early signal attenuation affects stability and accuracy of the continuous glucose monitoring apparatus (CGM) such that a user cannot obtain accurate glucose data at an early stage of using the CGM.

The present invention employs technical solutions as follows:

In an aspect of the present invention, the present invention provides a compensation method for a continuous glucose monitoring apparatus. The method includes: acquiring a real-time glucose value of a user continuously;

determining a user type and a glucose trend type according to the real-time glucose value, where the user type includes at least a first user type and a second user type;

determining, based on the real-time glucose value, whether a compensation start condition is satisfied; starting a compensation algorithm if the compensation start condition is satisfied; determining a compensation formula based on the real-time glucose value, the glucose trend type, and the first user type or the second user type; and obtaining, based on the real-time glucose value and the compensation formula, a compensated glucose value until a compensation stop condition is satisfied.

The term real-time refers to an operation of real-time systems that can reliably deliver specific results within a predetermined period of time, for example, in a fixed or variable time interval. In a practicable implementation, the compensation formula includes a first compensation coefficient and a second compensation coefficient.

In a practicable implementation, the first compensation coefficient is determined according to a concentration interval within which the real-time glucose value falls and the glucose trend type. The first compensation coefficient is substituted into an initial formula, and an intermediate formula is obtained.

The second compensation coefficient is determined based on an initial compensation value obtained by using the intermediate formula. The second compensation coefficient is substituted into the intermediate formula, and the compensation formula is obtained.

In a practicable implementation, when the initial compensation value obtained based on the intermediate formula is less than or equal to a first set threshold, the second compensation coefficient is determined according to a concentration interval within which the initial compensation value falls. When the initial compensation value is not less than the first set threshold, the intermediate formula is taken as the compensation formula.

In a practicable implementation, the compensated glucose value is dependent on the first compensation coefficient, the second compensation coefficient, an in-vivo sensitivity of the continuous glucose monitor and an operating current of the continuous glucose monitoring apparatus.

In a practicable implementation, the compensation formula is as follows: $GLU_{ESA} = \frac{I_w}{K_s(1+\alpha)} + \beta$ .

Specifically, $GLU_{ESA}$ is the compensated glucose value, $\alpha$ is the first compensation coefficient and a non-positive number, $\beta$ is the second compensation coefficient and a non-negative number, $I_w$ is an operating current of the continuous glucose monitoring apparatus, $K_s$ is in-vivo sensitivity of the continuous glucose monitoring apparatus, and $\frac{I_w}{K_s}$ is the real-time glucose value.

In a practicable implementation, the glucose trend type is related to a decline speed of the real-time glucose value, in other words, a descent rate of change of the real-time glucose value. The glucose trend type may comprises a gradual decline and a sharp decline.

In a practicable implementation, if a decline speed of the real-time glucose value is less than a first speed threshold and greater than a second speed threshold, the glucose trend type is a gradual decline.

If the decline speed of the real-time glucose value is less than the second speed threshold, the glucose trend type is a sharp decline. The first speed threshold and the second speed threshold are both negative numbers.

In a practicable implementation, the user type is related to a glucose fluctuation of the user. Preferably, the first user type has a smaller glucose fluctuation than the second user type.

In a practicable implementation, during preset compensation time after at least a part of the continuous glucose monitoring apparatus is implanted in a human body, if a maximum value of the real-time glucose value never exceeds the second set threshold, the user type is the first user type. If the maximum value of the real-time glucose value exceeds the second set threshold, the user type is the second user type.

In a practicable implementation, compensation start condition of the first user type includes: the real-time glucose value is less than a first start threshold, and the glucose trend type is a gradual decline or a sharp decline. Compensation start condition of the second user type includes: the real-time glucose value is less than a second start threshold, and the glucose trend type is the gradual decline or the sharp decline. The first start threshold is greater than the second start threshold.

In a practicable implementation, the compensation stop condition is as follows:

time during which at least a part of the continuous glucose monitoring apparatus is implanted in the human body reaches the preset compensation time; or
the real-time glucose value exceeds a third set threshold; or
when a minimum value of the real-time glucose values is greater than or equal to a fourth set threshold, compensation for the continuous glucose monitoring apparatus of the first user type is stopped; when the minimum value of the real-time glucose value is greater than or equal to a fifth set threshold, compensation for the continuous glucose monitoring apparatus of the second user type is stopped; and the fourth set threshold is greater than the fifth set threshold.

In a practicable implementation, in a situation that a current user type is the first user type,

if the real-time glucose value falls within a first threshold interval and the glucose trend type is a sharp decline, first compensation coefficient $\alpha$ is a first value depending on the real-time glucose value, for example as follows: $\alpha = 0.1 \times GLU$ - 0.55, where $GLU$ is the real-time glucose value; and
if the real-time glucose value falls within the first threshold interval and the glucose trend type is a gradual decline, or the real-time glucose value falls within a second threshold interval and the glucose trend type is the gradual decline or the sharp decline, first compensation coefficient $\alpha$ is a second value depending on the real-time glucose value, for example as follows: $\alpha = 0.1 \times GLU$ - 0.5, where a minimum value of the first threshold interval is greater than a maximum value of the second threshold interval.

In a situation that the current user type is the second user type,

if the real-time glucose value falls within a third threshold interval and the glucose trend type is the sharp decline, first compensation coefficient $\alpha$ is a third value depending on the real-time glucose value, for example as follows: $\alpha = 0.05 \times GLU$ - 0.29; and

if the real-time glucose value falls within the third threshold interval and the glucose trend type is the gradual decline, or the real-time glucose value falls within the second threshold interval and the glucose trend type is the gradual decline or the sharp decline, first compensation coefficient $\alpha$ is a fourth value depending on the real-time glucose value, for example as follows: $\alpha = 0.05 \times GLU - 0.25$, where a minimum value of the third threshold interval is greater than a maximum value of the fourth threshold interval.

Preferably, the first value, the second value, the third value and the fourth value of the first compensation coefficient $\alpha$ are different from each other.

In a practicable implementation, the first compensation coefficient ($\alpha$) and the second compensation coefficient ($\beta$) in the compensation formula are sequentially adjusted according to the real-time glucose value.

In another aspect of the present invention, the present invention further provides an early signal attenuation (ESA) compensation device for a CGM. The device includes: at least one processor; and a memory in communication connection to the at least one processor. The memory stores instructions which, when executed by the at least one processor, cause the at least one processor to carry out the compensation method for a continuous glucose monitoring apparatus.

The present invention provides further a continuous glucose monitoring apparatus comprising the compensation device. The continuous glucose monitoring apparatus may comprise a first part that can be partially implanted in a human body for acquiring the glucose value and a remote part that is separated from the first part. The compensation device may be disposed in the first part or the remote part of the continuous glucose monitoring apparatus.

Compared with the prior art, a compensation method and device for a continuous glucose monitoring apparatus provided in embodiments of the present invention have beneficial effects as follows:
In the present invention, a real-time glucose value of a user is continuously acquired by a continuous glucose monitoring apparatus (CGM), a user type and a glucose trend type are classified according to an actual glucose value, and further a compensation start condition is determined. After it is determined that ESA occurs on the user, in other words, the compensation start condition is satisfied, different compensation formulas are generated for users of different types and different glucose trend types based on a real-time glucose value. Moreover, glucose data outputted by the CGM are corrected and compensated by using the compensation formula, such that more accurate glucose data are obtained. Moreover, a compensation operation is timely stopped after a compensation stop condition is satisfied, such that redundant ESA compensation is avoided. According to the method provided in the present invention, through the above settings, data that is more accurate and closer to the actual glucose value can be obtained. An impact of the ESA on accuracy of the CGM is reduced.

## BRIEF DESCRIPTION OF THE DRAWINGS

To more clearly describe the technical solutions in embodiments of the present invention or in the prior art, accompanying drawings required to be used in descriptions of the embodiments or the prior art are briefly introduced. Apparently, accompanying drawings in the following descriptions are merely some embodiments of the present invention. A person of ordinary skill in the art can further derive other accompanying drawings according to these accompanying drawings. In the accompanying drawings:

FIG. 1 is a flowchart of a compensation method for a continuous glucose monitoring apparatus according to an embodiment of the present invention;
FIG. 2 is a specific flowchart of a compensation method for a continuous glucose monitoring apparatus according to an embodiment of the present invention; and
FIG. 3 is a schematic structural diagram of a compensation device for a continuous glucose monitoring apparatus according to an embodiment of the present invention.

## DETAILED DESCRIPTION

To enable a person skilled in the art to better understand the technical solution of the present invention, the technical solutions in the embodiments of the present invention are clearly and completely described in combination with the accompanying drawings in the embodiments of the present invention. Apparently, the described embodiments are merely some embodiments rather than all embodiments of the present invention. All other embodiments obtained by a person of ordinary skill in the art based on the embodiments of the description without making creative efforts shall fall within the scope of protection of the present invention.

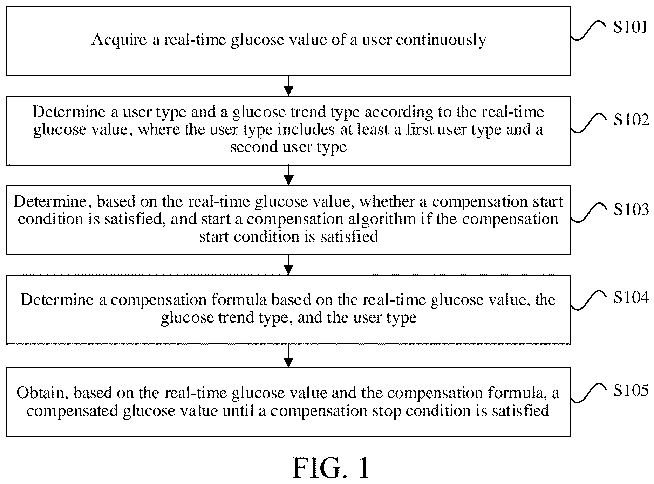

An embodiment of the present invention provides a compensation method for a continuous glucose monitoring apparatus. As shown in FIG. 1, the compensation method for a continuous glucose monitoring apparatus specifically includes steps S101 to S105 as follows:

S101: Acquire a real-time glucose value of a user continuously.

Specifically, in a normal situation, an operating current of a continuous glucose monitoring apparatus (CGM) and a glucose concentration of a human body have a linear directly proportional corresponding relationship. A conversion coefficient between the operating current and the glucose concentration is defined as in-vivo sensitivity. A basic principle formula of the CGM is as follows: $GLU = \dfrac{I_w}{K_s}$. Specifically, GLU is a glucose concentration, that is, a glucose value, of a human body; $I_w$ is an operating current of the CGM; and $K_s$ is in-vivo sensitivity of the CGM.

In the CGM, $K_s$ is generally set as a constant according to measurement and experience on a production line. Thus, by measuring a change in $I_w$, a corresponding change in glucose of the human body can be estimated.

Since the ESA mostly occurs in the first several days when a user starts to wear a CGM, after the user starts to wear the CGM, a real-time glucose value of the user is continuously acquired by the CGM. The real-time glucose value is a value computed according to the above basic principle formula. When the ESA occurs, the real-time glucose value does not represent an actual glucose value of the user.

S102: Determine a user type and a glucose trend type according to the real-time glucose value, where the user type includes at least a first user type and a second user type.

Specifically, during preset compensation time after the at least a part of the CGM is implanted in a human body, if a maximum value of the real-time glucose value always does not exceed the second set threshold, the user type is the first user type. If the maximum value of the real-time glucose value exceeds the second set threshold, the user type is the second user type.

As a practicable implementation, during preset compensation time starting from time when the at least a part of the CGM is implanted in the user, the real-time glucose value is continuously computed according to a basic principle formula of the CGM. Moreover, a maximum value and a minimum value of historical glucose values before the current moment are obtained. If the maximum value never exceeds the set threshold (that is, the second set threshold), it is deemed that a glucose fluctuation of the user is relatively small, and the user is determined as the first user type. If the maximum value exceeds the set threshold (that is, the second set threshold), it is deemed that the glucose fluctuation of the user is relatively high, and the user is determined as the second user type.

A value range of the preset compensation time is 2 days to 4 days, and is preferably 3 days. A value range of the second set threshold is [8 mmol/L, 11 mmol/L], and is preferably 9 mmol/L.

In an embodiment, within three days (that is, during the preset compensation time) when the user uses the CGM, the ESA compensation algorithm counts, in real time, the maximum value and the minimum value of the real-time glucose value computed according to the CGM basic principle formula. Assuming that the current moment is moment t (t ≤ 3 days), a maximum value and a minimum value of the real-time glucose value of the user in a time period from 0 to t are counted. If the maximum value is 8 mmol/L, and does not exceed the second set threshold 9 mmol/L, it is determined that the glucose fluctuation of the user is relatively small in the time period from 0 to t. The user is set as an A-type user (that is, the first user type).

If the real-time glucose value reaches 9.5 mmol/L at moment (t+1), the maximum glucose value in a time period from 0 to (t+1) is 9.5 mmol/L. In this case, the maximum glucose value exceeds the second set threshold 9 mmol/L. It indicates that the glucose fluctuation of the user in the time period from 0 to (t+1) is relatively large. The user becomes a B-type user (that is, the second user type).

It can be known based on the above embodiments that the user type of the user during the preset compensation time is not changeless, but changes according to a constant change of the real-time glucose value. The user may be the first user type at a previous second, and becomes the second user type at a next second. Thus, in a compensation process, a user type change of the user is required to be always monitored, such that different compensation formula adjustment methods are used.

Further, during the preset compensation time, the real-time glucose value is monitored to compute a real-time decline trend and a decline speed of glucose. If a decline speed of the real-time glucose value is less than a first speed threshold and greater than a second speed threshold, the glucose trend type is a gradual decline. If the decline speed of the real-time glucose value is less than the second speed threshold, the glucose trend type is a sharp decline. The first speed threshold and the second speed threshold are both negative numbers.

As a practicable implementation, the ESA compensation algorithm continuously computes a change trend of a glucose value estimated by using a CGM basic principle formula, to determine whether the glucose value estimated within each observation window period gradually or sharply declines. The glucose change trend may be computed through a linear regression method or a numerical difference method.

A value range of the observation window period is 10 min to 15 min, and is preferably 12 min. A value range of the first speed threshold is [-0.04 mmol/(L·min), -0.02 mmol/(L·min)], and is preferably -0.03 mmol/(L·min). A value range of the second speed threshold is [-0.07 mmol/(L·min), -0.04 mmol/(L·min)], and is preferably -0.06 mmol/(L·min).

In an embodiment, the observation window period is set as 12 min. A decline speed of the real-time glucose value is computed every 12 min from moment 0. If the glucose decline speed within the current observation window period is less than a first speed threshold -0.03 mmol/(L•min) and greater than a second speed threshold -0.06 mmol/(L•min), it is determined that the glucose trend type within the current observation window period is a gradual decline. If the glucose decline speed within the current observation window period is less than a second speed threshold -0.06 mmol/(L•min), it is determined that the glucose trend type within the current observation window period is a sharp decline. If the glucose decline speed is greater than the first speed threshold -0.03 mmol/(L•min), it is deemed that the current glucose decline speed is within a normal fluctuation range, and is not deemed as an abnormal decline.

It should be noted that the values used in the above embodiments are all illustrative, and are not used for limiting actually used values in the solution. Actually used data may be flexibly set according to an actual situation or expert experience.

In the present invention, a real-time glucose value is continuously monitored in the first several days when a user uses a CGM. Moreover, a series of data analyses are performed on the obtained real-time glucose value, such that a user type and a glucose trend type are determined. Thus, different compensation coefficient adjustment rules are subsequently used according to different user types and different glucose trend types, to adapt to individual differences of users. Further, glucose values after compensation are closer to actual glucose values of different users.

S103: Determine, based on the real-time glucose value, whether a compensation start condition is satisfied, and start a compensation algorithm if the compensation start condition is satisfied.

Specifically, compensation start conditions of the first user type are as follows: the real-time glucose value is less than a first start threshold, and the glucose trend type is a gradual decline or a sharp decline. Compensation start conditions of the second user type are as follows: the real-time glucose value is less than a second start threshold, and the glucose trend type is the gradual decline or the sharp decline. The first start threshold is greater than the second start threshold.

As a practicable implementation, if the current user is of the first user type at the current moment, that is, the glucose fluctuation is relatively small, if the real-time glucose value estimated by using the CGM basic principle formula is less than the first start threshold, and the glucose trend type is a gradual decline or a sharp decline, the ESA compensation algorithm is started. In a situation that the current user is of the second user type at the current moment, that is, the glucose fluctuation is relatively large, if the real-time glucose value estimated by using the CGM basic principle formula is less than the second start threshold, and the glucose trend type is a gradual decline or a sharp decline, the ESA compensation algorithm is started. Since the glucose fluctuation of the second user type is relatively large and a relatively small glucose value for the first-type user may be a normal value for the second-type user, a relatively small start threshold is required to be set for the second-type user, to adapt to characteristics of this type of user. Thus, the second start threshold is set to be less than the first start threshold.

A value range of the first start threshold is [3.5 mmol/L, 5 mmol/L], and is preferably 4.4 mmol/L. A value range of the second start threshold is [2 mmol/L, 3.5 mmol/L), and is preferably 3.0 mmol/L.

In an embodiment, when the real-time glucose value of the A-type user is less than the first start threshold 4.4 mmol/L and is gradually or sharply declining, the ESA compensation algorithm is started to perform glucose compensation for the A-type user. When the real-time glucose value of the B-type user is less than the second start threshold 3.0 mmol/L and is gradually or sharply declining, the ESA compensation algorithm is started to perform glucose compensation for the B-type user.

It should be noted that the start threshold values listed in the above embodiments are all illustrative, and are not used for limiting actual start threshold values in this solution. Actually used data may be flexibly set according to an actual situation or expert experience.

In the present invention, compensation start conditions of different user types and different glucose decline trends are specified according to signs indicating that the ESA may occur on different types of users. ESA compensation is started only when the real-time glucose value of the user is less than a particular value and the decline speed is also less than a particular value. A situation that mistaken compensation is performed when no ESA occurs on the CGM is avoided. Accuracy of data at an early stage of using the CGM by the user is ensured.

S104: Determine a compensation formula based on the real-time glucose value, the glucose trend type, and the user type.

Specifically, first, in the present invention, an ESA compensation formula is self-defined according to a basic principle formula of the CGM as follows: $GLU_{ESA} = \frac{I_w}{K_s(1+\alpha)} + \beta$ . Specifically, $GLU_{ESA}$ is the compensated glucose value, $I_w$ is an operating current of the continuous glucose monitoring apparatus, $K_s$ is the in-vivo sensitivity of the continuous glucose monitoring apparatus, and $\frac{I_w}{K_s}$ is a basic principle formula of the CGM, and represents a real-time glucose value estimated by the CGM. Moreover, $\alpha$ is a first compensation coefficient and a non-positive number, and is used for reducing $K_s$ to improve an estimated glucose value; and $\beta$ is a second compensation coefficient and a non-negative number, and is used for making up for a limitation that in some scenarios, it is not enough to increase a glucose value to an expected range only by using $\alpha$.

Further, first compensation coefficient $\alpha$ is determined according to a concentration interval within which the real-time glucose value falls and the glucose trend type. Then, first compensation coefficient $\alpha$ is substituted into an initial formula, and an intermediate formula is obtained. In the initial formula, $\alpha$ and $\beta$ both are 0. In this case, the initial formula is equivalent to $GLU_{ESA} = \frac{I_w}{K_s}$. After the value of first compensation coefficient $\alpha$ is determined, an obtained intermediate formula is equivalent to $GLU_{ESA} = \frac{I_w}{K_s(1+\alpha)}$.

Further, second compensation coefficient $\beta$ is determined based on an initial compensation value obtained by using the intermediate formula. Then, second compensation coefficient $\beta$ is substituted into the intermediate formula, and a final compensation formula is obtained. After the value of second compensation coefficient $\beta$ is determined, an obtained compensation formula is a final complete compensation formula as follows: $GLU_{ESA} = \frac{I_w}{K_s(1+\alpha)} + \beta$.

As a practicable implementation, when the initial compensation value obtained based on the intermediate formula is less than or equal to a first set threshold, the second compensation coefficient is determined according to a concentration interval within which the initial compensation value falls. When the initial compensation value is not less than or not equal to the first set threshold, the intermediate formula is taken as a final compensation formula. A value range of the first set threshold is [1.7 mmol/L, 3.5 mmol/L], and is preferably 3.5 mmol/L.

Specifically, in a situation that the current user type is the first user type, if the real-time glucose value falls within the first threshold interval and the glucose trend type is a sharp decline, first compensation coefficient $\alpha$ is as follows: $\alpha = 0.1 \times GLU$ - 0.55, where $GLU$ is the real-time glucose value. If the real-time glucose value falls within the first threshold interval and the glucose trend type is a gradual decline, or the real-time glucose value falls within a second threshold interval and the glucose trend type is the gradual decline or the sharp decline, first compensation coefficient $\alpha$ is as follows: $\alpha = 0.1 \times GLU$ - 0.5, where a minimum value of the first threshold interval is greater than a maximum value of the second threshold interval.

Then, computed first compensation coefficient $\alpha$ is substituted into a self-defined ESA compensation formula. An initial compensation value at this time is computed. If the initial compensation value is less than or equal to the first set threshold, second compensation coefficient $\beta$ is continued to be adjusted according to an interval of the initial compensation value.

The first threshold interval is preferably [3.5 mmol/L, 4.4 mmol/L]. The second threshold interval is preferably less than 3.5 mmol/L.

In an embodiment, for a first user type of which a glucose fluctuation is relatively small, an adjustment strategy of first compensation coefficient $\alpha$ is shown in Table 1.

Table 1

| Real-time glucose value | Glucose trend type | Adjustment strategy of $\alpha$ |
|---|---|---|
| At an interval of [3.5 mmol/L, 4.4 mmol/L] | Sharp decline | $\alpha = 0.1 \times GLU$ - 0.55 |
| At an interval of [3.5 mmol/L, 4.4 mmol/L] | Gradual decline | $\alpha = 0.1 \times GLU$ - 0.5 |
| Less than 3.5 mmol/L | Gradual decline or sharp decline | $\alpha = 0.1 \times GLU$ - 0.5 |
| Other ranges | / | Not adjusted |

First compensation coefficient $\alpha$ is computed according to an adjustment strategy in Table 1, and then substituted into a self-defined ESA compensation formula. An initial compensation value is computed.

In a further embodiment, if the initial compensation value is less than or equal to first set threshold 3.5 mmol/L, an adjustment strategy of second compensation coefficient $\beta$ is shown in Table 2.

Table 2

| Initial compensation value | Adjustment strategy of $\beta$ |
|---|---|
| Less than 3.0 mmol/L | $\beta$ increased by 0.5 |
| At an interval of [3.0 mmol/L, 3.5 mmol/L] | $\beta$ increased by 0.25 |
| Other ranges | Not adjusted |

An adjustment range of first compensation coefficient $\alpha$ is limited between -0.25 to 0. An adjustment range of second compensation coefficient $\beta$ is limited between 0 to 2.5. So far, an ESA compensation coefficient of the first-type user of which a glucose fluctuation is small is adjusted.

Further, in a situation that the current user type is the second user type, if the real-time glucose value falls within a third threshold interval and the glucose trend type is the sharp decline, first compensation coefficient $\alpha$ is as follows: $\alpha = 0.05 \times GLU$ - 0.29. If the real-time glucose value falls within the third threshold interval and the glucose trend type is the gradual decline, or the real-time glucose value falls within the second threshold interval and the glucose trend type is the gradual decline or the sharp decline, first compensation coefficient $\alpha$ is as follows: $\alpha = 0.05 \times GLU$ - 0.25, where a minimum value of the third threshold interval is greater than a maximum value of the fourth threshold interval.

Then, computed first compensation coefficient $\alpha$ is substituted into a self-defined ESA compensation formula. An initial compensation value at this time is computed. If the initial compensation value is less than or equal to the first set threshold, second compensation coefficient $\beta$ is continued to be adjusted according to an interval of the initial compensation value.

The third threshold interval is preferably [2.2 mmol/L, 3.0 mmol/L], and the second threshold interval is preferably [1.7 mmol/L, 2.2 mmol/L].

In an embodiment, for the second user type of which glucose fluctuation is relatively large, an adjustment strategy of first compensation coefficient $\alpha$ is shown in Table 3.

Table 3

| Real-time glucose value | Glucose trend type | Adjustment strategy of $\alpha$ |
|---|---|---|
| At an interval of [2.2 mmol/L, 3.0 mmol/L] | Sharp decline | $\alpha = 0.05 \times GLU$ - 0.29 |
| At an interval of [2.2 mmol/L, 3.0 mmol/L] | Gradual decline | $\alpha = 0.05 \times GLU$ - 0.25 |
| Less than 2.2 mmol/L | Gradual decline or sharp decline | $\alpha = 0.05 \times GLU$ - 0.25 |
| Other ranges | / | Not adjusted |

First compensation coefficient $\alpha$ is computed according to an adjustment strategy in Table 1, and then substituted into a self-defined ESA compensation formula. An initial compensation value is computed.

In a further embodiment, if the initial compensation value is less than or equal to first set threshold 3.5 mmol/L, an adjustment strategy of second compensation coefficient $\beta$ is shown in Table 4.

Table 4

| Initial compensation value | Adjustment strategy of $\beta$ |
|---|---|
| Less than 2.2 mmol/L | $\beta$ increased by 0.8 |
| At an interval of [2.2 mmol/L, 3.0 mmol/L] | $\beta$ increased by 0.5 |
| At an interval of [3.0 mmol/L, 3.5 mmol/L] | $\beta$ increased by 0.2 |
| Other ranges | Not adjusted |

An adjustment range of first compensation coefficient $\alpha$ is limited between -0.25 to 0. An adjustment range of second compensation coefficient $\beta$ is limited between 0 to 2.5. So far, an ESA compensation coefficient of the second-type user of which a glucose fluctuation is large is adjusted.

In the present invention, an ESA compensation algorithm and an ESA compensation formula are self-defined based on a basic principle of a continuous glucose monitoring apparatus (CGM). Two compensation coefficients in the ESA compensation formula corresponding to different user types are sequentially adjusted according to a real-time glucose value of a user, to perform a compensation adjustment on a deviation glucose value outputted by the CGM. Thus, more accurate data that adapts to an individual difference of a user and is closer to an actual glucose value are outputted.

S105: Obtain, based on the real-time glucose value and the compensation formula, a compensated glucose value until a compensation stop condition is satisfied.

Specifically, the adjusted compensation coefficient obtained in the previous step is substituted into the ESA compensation formula, to continuously compensate for the real-time glucose value obtained by the CGM.

Further, compensation stop conditions of an ESA compensation algorithm are as follows:

(1) time during which at least a part of the continuous glucose monitoring apparatus is implanted in the human body reaches the preset compensation time;

(2) the real-time glucose value exceeds a third set threshold;

(3) when a minimum value of the real-time glucose values is greater than or equal to a fourth set threshold, compensation for the continuous glucose monitoring apparatus of the first user type is stopped; when the minimum value of the real-time glucose value is greater than or equal to a fifth set threshold, compensation for the continuous glucose monitoring apparatus of the second user type is stopped; and the fourth set threshold is greater than the fifth set threshold.

As a practicable implementation, since the ESA more frequently occurs in the first several days when a user starts to wear the CGM, the ESA compensation algorithm takes effect in the first several days when the CGM starts to be used. After the preset compensation time expires, ESA compensation is no longer performed.

During the preset compensation time, if in an ESA compensation process, the real-time glucose value computed by using the CGM basic principle formula exceeds the third set threshold, the ESA compensation is stopped, and compensation coefficients $\alpha$ and $\beta$ are reset to be 0.

During the preset compensation time, in an ESA compensation process, if a minimum value of a real-time glucose value of a first-type user within set duration is not less than a fourth set threshold, it is deemed that no ESA occurs on the user, and subsequently, ESA compensation is no longer performed. If a minimum value of a real-time glucose value of a second-type user within set duration is not less than a fifth set threshold, it is deemed that no ESA occurs on the user, and subsequently, no ESA compensation is performed. Compared with the first-type user, the second-type user has a larger glucose fluctuation. Thus, a historical minimum glucose value of the second-type user is less than that of the first-type user. Thus, the fifth set threshold is set to be less than the fourth set threshold.

A value range of the third set threshold is [4 mmol/L, 6 mmol/L], and is preferably 5 mmol/L. A value range of the fourth preset threshold is [3.5 mmol/L, 4 mmol/L), and is preferably 3.9 mmol/L. A value range of the fifth preset threshold is [2.5 mmol/L, 3.5 mmol/L), and is preferably 3 mmol/L. A value range of the set duration is 15 h to 36 h.

In an embodiment, after the time during which the user wears the CGM reaches the preset compensation time for three days, ESA compensation is no longer performed.

Or within 3 days, if in an ESA compensation process, the real-time glucose value exceeds the third set threshold 5 mmol/L, the ESA compensation is stopped, and compensation coefficients $\alpha$ and $\beta$ are reset to be 0.

Or within 3 days, if in an ESA compensation process, a minimum value of a real-time glucose value of a first-type user within set duration 15 h is always not less than a fourth set threshold 3.9 mmol/L, it is deemed that no ESA occurs on the user, and ESA compensation is stopped. If the minimum value of the real-time glucose value of the second-type user during the set duration 15 h is always not less than the fifth set threshold 3.0 mmol/L, it is deemed that no ESA occurs on the user, and ESA compensation is stopped.

It should be noted that each specific value listed in the above embodiment may be flexibly adjusted within a particular range. For example, 15 h may be adjusted to 36 h, and the threshold may also be adjusted to another value, as long as a value relationship among the thresholds is satisfied. Thus, values in the above embodiments are not used for limiting this solution, but are merely illustrative descriptions for clearly describing this solution.

In the present invention, a plurality of detailed compensation stop conditions are specified according to a real-time glucose value obtained by a CGM. Thus, redundant compensation operations are avoided when ESA compensation is not required to be performed. Accuracy of an outputted glucose value is ensured in a plurality of aspects.

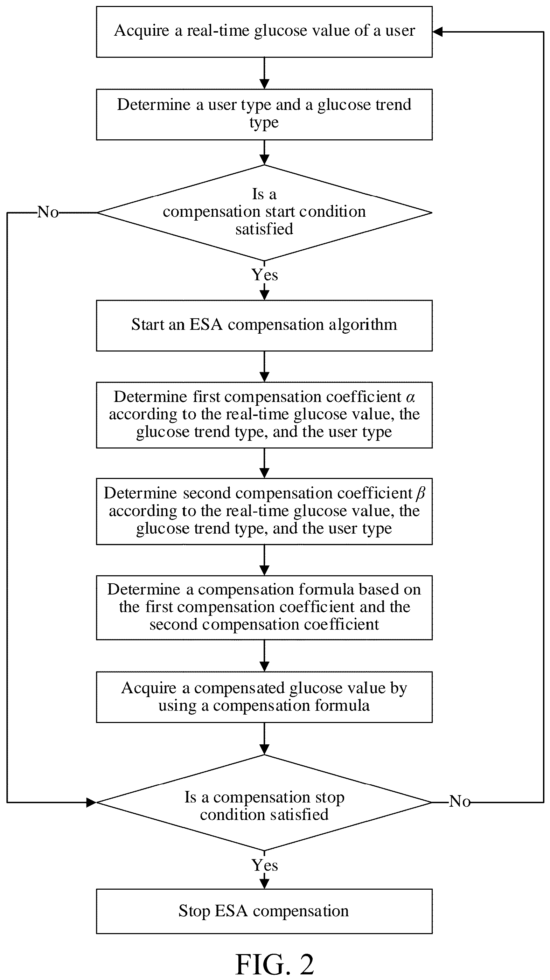

It should be noted that the entire solution of the present invention is an iterative process. To clearly describe the iterative process, a detailed description is made with FIG. 2 as an embodiment.

FIG. 2 is a specific flowchart of a compensation method for a continuous glucose monitoring apparatus according to an embodiment of the present invention. As shown in FIG. 2, a real-time glucose value of a user at a current moment is first obtained by a CGM. Then, a user type and a glucose trend type are determined according to the real-time glucose value. Further, whether a compensation start condition is satisfied at the current moment is determined according to the user type and the glucose trend type. If the compensation stop condition is not satisfied, whether the compensation stop condition is satisfied is continued to be determined. If the compensation stop condition is satisfied, it is determined that the ESA no longer occurs on the user, and the algorithm is directly ended. If the compensation stop condition is not satisfied, a real-time glucose value at a next moment is continued to be obtained, and ESA compensation is temporarily not started. If the start condition is satisfied, the ESA compensation algorithm is started, and first compensation coefficient $\alpha$ is determined according to the real-time glucose value, the glucose trend type, and the user type. Then, second compensation coefficient $\beta$ is determined according to the initial compensation value and the user type, and the compensation formula is obtained. Further, a compensation glucose value is obtained by using a compensation formula. Finally, whether a compensation stop condition is currently satisfied is determined according to parameters such as the real-time glucose value and CGM usage time. If the compensation stop condition is satisfied, ESA compensation is stopped. If the compensation stop condition is not satisfied, a real-time glucose value at a next moment is continued to be obtained. An iteration loop of the above process is performed.

In the present invention, a real-time glucose value of a user is continuously acquired by a continuous glucose monitoring apparatus (CGM), a user type and a glucose trend type are classified according to an actual glucose value, and further a compensation start condition is determined. After it is determined that ESA occurs on the user, different compensation formulas are generated for users of different types and different glucose trend types based on a real-time glucose value. Moreover, glucose data outputted by the CGM are corrected and compensated by using the compensation formula, such that more accurate glucose data are obtained. The present invention further provides a detailed compensation start condition and a detailed compensation stop condition. Thus, it can be ensured that no interference is performed when ESA compensation is not required to be performed. Redundant ESA compensation is avoided. The method provided in the present invention can obtain data that is more accurate and closer to an actual glucose value, and is applicable to users having different glucose fluctuation degrees, which cannot be generalized. Impacts of the ESA on stability and accuracy of the CGM are greatly reduced.

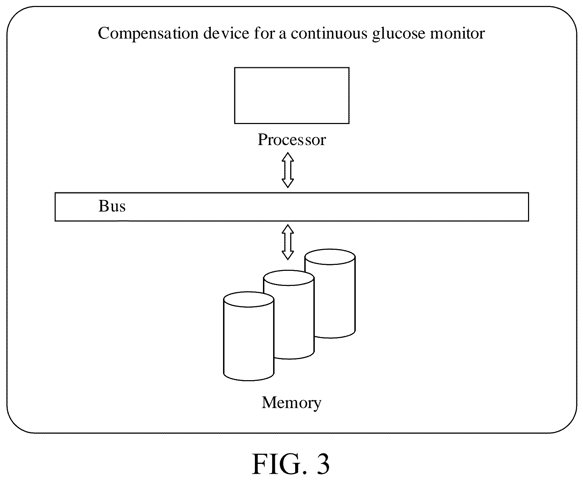

In addition, an embodiment of the present invention further provides a compensation device for a continuous glucose monitoring apparatus. As shown in FIG. 3, the compensation device for a continuous glucose monitoring apparatus specifically includes:

at least one processor; and a memory in communication connection to the at least one processor.

The memory stores instructions capable of being executed by the at least one processor, and the at least one processor is capable of performing steps as follows:

a real-time glucose value of a user is acquired continuously;
a user type and a glucose trend type are determined according to the real-time glucose value, where the user type includes at least a first user type and a second user type;
based on the real-time glucose value, whether a compensation start condition is satisfied is determined; a compensation algorithm is started if the compensation start condition is satisfied; a compensation formula is determined based on the real-time glucose value, the glucose trend type, and the user type; and based on the real-time glucose value and the compensation formula, a compensated glucose value is obtained until a compensation stop condition is satisfied.

The embodiments of the present invention are all described in a progressive manner. Same or similar parts among the embodiments can be obtained with reference to each other. Each embodiment focuses on a difference from other embodiments. Especially, an apparatus embodiment, a device embodiment, a non-volatile computer storage medium embodiment are basically similar to a method embodiment, and thus are briefly described. Related parts can be obtained with reference to some descriptions of the method embodiment.

Particular embodiments of the present invention are described above. In addition, the processes depicted in the accompanying drawings are not certainly performed in a particular order or consecutive order to achieve an expected result. In some implementations, multitasking and parallel processing may be practicable or beneficial.

The above descriptions are merely embodiments of the present invention, but are not intended to limit the present invention. A person skilled in the art can make various changes and variations to the embodiments of the present invention.

## Claims

1. A compensation method for a continuous glucose monitoring apparatus, comprising:

   acquiring (S101) a real-time glucose value of a user continuously;
   determining (S102) a user type and a glucose trend type according to the real-time glucose value, wherein the user type comprises at least a first user type and a second user type;
   determining (S103), based on the real-time glucose value, whether a compensation start condition is satisfied; starting a compensation algorithm if the compensation start condition is satisfied; determining (S104) a compensation formula based on the real-time glucose value, the glucose trend type, and the first user type or the second user type; and obtaining (S105), based on the real-time glucose value and the compensation formula, a compensated glucose value until a compensation stop condition is satisfied.

2. The compensation method for a continuous glucose monitoring apparatus according to claim 1, wherein the compensation formula comprises a first compensation coefficient ($\alpha$) and a second compensation coefficient ($\beta$).

3. The compensation method for a continuous glucose monitoring apparatus according to claim 2, the first compensation coefficient ($\alpha$) is determined according to a concentration interval within which the real-time glucose value falls and the glucose trend type; the first compensation coefficient ($\alpha$) is substituted into an initial formula, and an intermediate formula is obtained;

the second compensation coefficient ($\beta$) is determined based on an initial compensation value obtained by using the intermediate formula; and the second compensation coefficient ($\beta$) is substituted into the intermediate formula, and the compensation formula is obtained.

4. The compensation method for a continuous glucose monitoring apparatus according to claim 3, wherein when the initial compensation value obtained based on the intermediate formula is less than or equal to a first set threshold, the second compensation coefficient is determined according to a concentration interval within which the initial compensation value falls; and when the initial compensation value is not less than the first set threshold, the intermediate formula is taken as the compensation formula.

5. The compensation method for a continuous glucose monitor according to any one of claims 2 to 4, wherein the compensated glucose value is dependent on the first compensation coefficient ($\alpha$), the second compensation coefficient ($\beta$), an operating current of the continuous glucose monitor ($I_w$) and an in-vivo sensitivity of the continuous glucose monitor ($K_s$).

6. The compensation method for a continuous glucose monitoring apparatus according to any one of claims 1 to 4, wherein the compensation formula is as follows: $GLU_{ESA} = \dfrac{I_w}{K_s(1+\alpha)} + \beta$ , wherein $GLU_{ESA}$ is the compensated glucose value, $\alpha$ is the first compensation coefficient and a non-positive number, $\beta$ is the second compensation coefficient and a non-negative number, $I_w$ is an operating current of the continuous glucose monitoring apparatus, $K_s$ is an in-vivo sensitivity of the continuous glucose monitoring apparatus, and $\dfrac{I_w}{K_s}$ is the real-time glucose value.

7. The compensation method for a continuous glucose monitoring apparatus according to any one of claims 1 to 6, wherein if a decline speed of the real-time glucose value is less than a first speed threshold and greater than a second speed threshold, determining that the glucose trend type is a gradual decline;
if the decline speed of the real-time glucose value is less than the second speed threshold, determining that the glucose trend type is a sharp decline; and the first speed threshold and the second speed threshold are both negative numbers.

8. The compensation method for a continuous glucose monitoring apparatus according to any one of claims 1 to 7, wherein during preset compensation time after at least a part of the continuous glucose monitoring apparatus is implanted in a human body, if a maximum value of the real-time glucose value never exceeds a second set threshold, determining that the user type is the first user type; and if the maximum value of the real-time glucose value exceeds the second set threshold, determining that the user type is the second user type.

9. The compensation method for a continuous glucose monitoring apparatus according to any one of claims 1 to 8, wherein the compensation start condition comprises a compensation start conditions of the first user type and a compensation start condition of the second user type, wherein the compensation start condition of the first user type includes: the real-time glucose value is less than a first start threshold, and the glucose trend type is a gradual decline or a sharp decline; the compensation start condition of the second user type includes: the real-time glucose value is less than a second start threshold, and the glucose trend type is the gradual decline or the sharp decline; and the first start threshold is greater than the second start threshold.

10. The compensation method for a continuous glucose monitoring apparatus according to any one of claims 1 to 9, wherein the compensation stop condition is as follows:

time during which the continuous glucose monitoring apparatus is implanted in the human body reaches the preset compensation time; or
the real-time glucose value exceeds a third set threshold; or
when a minimum value of the real-time glucose values is greater than or equal to a fourth set threshold, compensation for the continuous glucose monitoring apparatus of the first user type is stopped; when the minimum value of the real-time glucose value is greater than or equal to a fifth set threshold, compensation for the continuous glucose monitoring apparatus of the second user type is stopped; and the fourth set threshold is greater than the fifth set threshold.

11. The compensation method for a continuous glucose monitoring apparatus according to any one of claims 2 to 10, wherein in a situation that a current user type is the first user type,

if the real-time glucose value falls within a first threshold interval and the glucose trend type is a sharp decline, the first compensation coefficient ($\alpha$) is a first value depending on the real-time glucose value; and

if the real-time glucose value falls within the first threshold interval and the glucose trend type is a gradual decline, or the real-time glucose value falls within a second threshold interval and the glucose trend type is the gradual decline or the sharp decline, the first compensation coefficient ($\alpha$) is a second value depending on the real-time glucose value, wherein a minimum value of the first threshold interval is greater than a maximum value of the second threshold interval; and

in a situation that the current user type is the second user type,

if the real-time glucose value falls within a third threshold interval and the glucose trend type is the sharp decline, the first compensation coefficient ($\alpha$) is a third value depending on the real-time glucose value; and

if the real-time glucose value falls within the third threshold interval and the glucose trend type is the gradual decline, or the real-time glucose value falls within the second threshold interval and the glucose trend type is the gradual decline or the sharp decline, the first compensation coefficient ($\alpha$) is a fourth value depending on the real-time glucose value, wherein a minimum value of the third threshold interval is greater than a maximum value of the fourth threshold interval, preferably wherein the first value, the second value, the third value and the fourth value of the first compensation coefficient ($\alpha$) are different from each other.

12. The compensation method for a continuous glucose monitoring apparatus according to claim 11, wherein the first value of the first compensation coefficient ($\alpha$) is as follows: $\alpha = 0.1 \times GLU - 0.55$, wherein $GLU$ is the real-time glucose value;

the second value of the first compensation coefficient ($\alpha$) is as follows: $\alpha = 0.1 \times GLU - 0.5$, wherein $GLU$ is the real-time glucose value;

the third value of the first compensation coefficient ($\alpha$) is as follows: $\alpha = 0.05 \times GLU - 0.29$, wherein $GLU$ is the real-time glucose value; and

the fourth value of the first compensation coefficient ($\alpha$) is as follows: $\alpha = 0.05 \times GLU - 0.25$, wherein $GLU$ is the real-time glucose value.

13. The compensation method for a continuous glucose monitoring apparatus according to any one of claims 2 to 12, wherein the first compensation coefficient ($\alpha$) and the second compensation coefficient ($\beta$) in the compensation formula are sequentially adjusted according to the real-time glucose value.

14. A compensation device for a continuous glucose monitoring apparatus, comprising:

at least one processor; and
a memory in communication connection to the at least one processor, wherein
the memory stores instructions which, when executed by the at least one processor, cause the at least one processor to carry out the compensation method for a continuous glucose monitor according to any one of claims 1 to 13.

15. A continuous glucose monitoring apparatus comprising the compensation device according to claim 14.

Acquire a real-time glucose value of a user continuously ⟋ S101

Determine a user type and a glucose trend type according to the real-time glucose value, where the user type includes at least a first user type and a second user type ⟋ S102

Determine, based on the real-time glucose value, whether a compensation start condition is satisfied, and start a compensation algorithm if the compensation start condition is satisfied ⟋ S103

Determine a compensation formula based on the real-time glucose value, the glucose trend type, and the user type ⟋ S104

Obtain, based on the real-time glucose value and the compensation formula, a compensated glucose value until a compensation stop condition is satisfied ⟋ S105

# FIG. 1

FIG. 2

Compensation device for a continuous glucose monitor

Processor

Bus

Memory

## FIG. 3

## EUROPEAN SEARCH REPORT

**Application Number**

EP 25 21 6878

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/277271 A1 (BUDIMAN ERWIN SATRYA [US] ET AL) 22 August 2024 (2024-08-22) * paragraphs [0006] - [0023], [0025] - [0028], [0055]; figure 1 * | 1-15 | INV. A61B5/145 |
| A | CA 2 665 323 A1 (ABBOTT DIABETES CARES INC [US]; ABBOTT DIABETES CARE INC [US]) 10 April 2008 (2008-04-10) * paragraphs [0011] - [0013]; figures 3,4,5 * | 1-15 | |
| A | JIA ZIRU ET AL: "Design of a Real-time Self-adjusting Calibration Algorithm to Improve the Accuracy of Continuous Blood Glucose Monitoring", APPLIED BIOCHEMISTRY AND BIOTECHNOLOGY HUMANA PRESS INC, NEW YORK, vol. 190, no. 4, 12 November 2019 (2019-11-12), pages 1163-1176, XP037134232, ISSN: 0273-2289, DOI: 10.1007/S12010-019-03142-7 [retrieved on 2019-11-12] * page 1166 - page 1169; figure 3 * | 1-15 | |

**TECHNICAL FIELDS SEARCHED (IPC)**

A61B

The present search report has been drawn up for all claims

| Place of search | Date of completion of the search | Examiner |
|---|---|---|
| Berlin | 27 March 2026 | Schatz, Veronika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 21 6878

27-03-2026

| Patent document cited in search report | | Publication date | Patent family member(s) | | Publication date |
|---|---|---|---|---|---|
| US 2024277271 | A1 | 22-08-2024 | EP | 2727530 A1 | 07-05-2014 |
| | | | US | 2014121480 A1 | 01-05-2014 |
| | | | US | 2017265790 A1 | 21-09-2017 |
| | | | US | 2018042531 A1 | 15-02-2018 |
| | | | US | 2019150802 A1 | 23-05-2019 |
| | | | US | 2022142518 A1 | 12-05-2022 |
| | | | US | 2023190156 A1 | 22-06-2023 |
| | | | US | 2024277271 A1 | 22-08-2024 |
| CA 2665323 | A1 | 10-04-2008 | CA | 2665323 A1 | 10-04-2008 |
| | | | EP | 2073691 A2 | 01-07-2009 |
| | | | US | 2008081977 A1 | 03-04-2008 |
| | | | US | 2010023291 A1 | 28-01-2010 |
| | | | US | 2013338454 A1 | 19-12-2013 |
| | | | US | 2016206233 A1 | 21-07-2016 |
| | | | US | 2017224258 A1 | 10-08-2017 |
| | | | US | 2018098721 A1 | 12-04-2018 |
| | | | WO | 2008042760 A2 | 10-04-2008 |